(19) Europäisches Patentamt
European Patent Office
Office européen des brevets

(11) Publication number: 0 253 650
A2

(12) # EUROPEAN PATENT APPLICATION

(21) Application number: 87306264.0

(22) Date of filing: 15.07.87

(51) Int. Cl.[4]: **C 07 D 333/68**
C 07 D 333/56,
C 07 D 409/04,
C 07 D 409/12, A 61 K 31/38

(30) Priority: 18.07.86 US 887019

(43) Date of publication of application:
20.01.88 Bulletin 88/03

(84) Designated Contracting States:
AT BE CH DE ES FR GB GR IT LI NL SE

(71) Applicant: ELI LILLY AND COMPANY
Lilly Corporate Center
Indianapolis Indiana 46285 (US)

(72) Inventor: Panetta, Jill Ann
195 Scranton Court
Zionsville Indiana 46077 (US)

(74) Representative: Tapping, Kenneth George et al
Erl Wood Manor
Windlesham Surrey, GU20 6PH (GB)

(54) **Improvements relating to benzothiophene derivatives.**

(57) This invention provides pharmaceutical formulations of certain benzothiophene derivatives and a method of using the derivatives or formulations for the treatment of asthma.

EP 0 253 650 A2

**Description**

IMPROVEMENTS RELATING TO BENZOTHIOPHENE DERIVATIVES

This invention relates to certain 2,5,6-trisubstituted-benzothiophene derivatives which are potent phosphodiesterase inhibitors and bronchodilators.

Recent studies have confirmed that theophylline is probably the most effective bronchodilator for the treatment of asthma that can be administered parenterally and orally. Unfortunately, the therapeutic range for theophylline is fairly narrow and its toxic effects stem largely from its action on the central nervous system, cardiovascular system, and gastro-intestinal tract. These undesirable pharmacological activities, as well as the bronchodilation effect, may be related to theophylline's ability to competitively inhibit phosphodiesterase (PDE), which is an enzyme that degrades cyclic AMP. It would therefore be desirable to discover selective inhibitors of cyclic AMP phosphodiesterase which possess a beneficial bronchodilation effect but lack most of the adverse effects of theophylline.

Several benzothiophene derivatives are known in the art. For example, 5,6-dialkoxybenzothiophene-2-carboxylic acids are taught to have antiasthmatic activity in U.S. Patent 4,552,891.

According to the invention there is provided a method for treating a mammal suffering from or susceptible to bronchoconstriction, particularly that derived from an asthmatic attack which comprises employing a compound represented by formula I

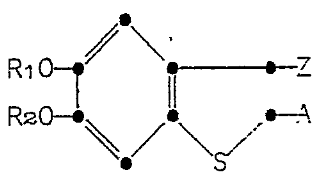

I

wherein each of $R_1$ and $R_2$ is independently $C_1$-$C_3$ alkyl, Z is hydrogen or methyl, and A is $-CH_2OH$, -CN, 5-tetrazolyl, or $-CONR_3R_4$, where $R_3$ and $R_4$ are each independently hydrogen or $C_1$-$C_3$ alkyl, or when one of $R_3$ and $R_4$ is hydrogen, the other of $R_3$ and $R_4$ is 5-tetrazolyl, or when taken together with the nitrogen atom to which they are attached form a piperidino, morpholino, or N-methyl piperazino ring;
or a pharmaceutically-acceptable salt.

According to a further aspect of the present invention there is provided a pharmaceutical formulation which comprises a compound of formula I as defined above associated with one or more pharmaceutically acceptable carriers or diluents therefor.

The preferred compounds to be employed in the method of this invention are those wherein $R_1$ and $R_2$ are each ethyl. Also preferred are compounds wherein Z is hydrogen. The most preferred compounds are 5,6-diethoxybenzothiophene-2-carboxamide and 5,6-diethoxybenzothiophene-2-methanol.

The term "$C_1$-$C_3$ alkyl" refers to straight and branched aliphatic radicals of one to three carbon atoms such as methyl, ethyl, propyl, and isopropyl.

This invention includes the use of pharmaceutically acceptable base addition salts of compounds containing a tetrazole substituent. Such salts include those derived from inorganic bases, such as ammonium, alkali and alkaline earth metal hydroxides, carbonates, bicarbonates, and the like, as well as salts derived from basic organic amines, such as aliphatic and aromatic amines, aliphatic diamines, hydroxy alkylamines, and the like. Such bases useful in preparing the salts of this invention thus include sodium hydroxide, sodium acetate, ammonium hydroxide, potassium carbonate, sodium bicarbonate, calcium hydroxide, methyl amine, diethyl amine, ethylenediamine, cyclohexylamine, ethanolamine, and the like. The potassium and sodium salt forms are particularly preferred.

Many of the compounds employed in this invention are known in the art and can be prepared according to standard methods. The following Scheme summarizes the preferred methods of preparation:

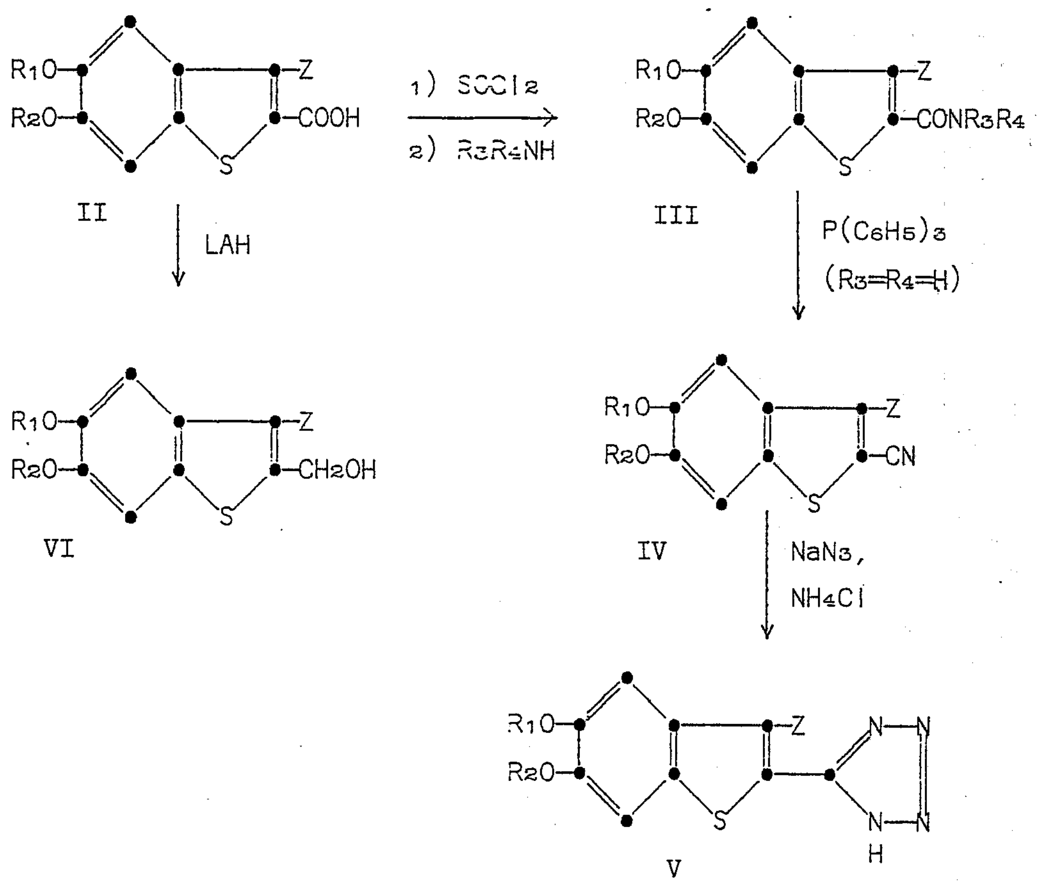


The carboxamides of formula III are prepared from the corresponding carboxylic acid II by first converting the acid into the acid halide, such as an acid chloride, upon heating with a thionyl halide, removing excess thionyl halide by evaporation, and then treating the crude acid halide with the appropriate amine $R_3R_4NH$. Conversion of the acid to the acid chloride is best achieved using thionyl chloride both as the reactant and solvent. Heating at reflux for 1 hour is sufficient to accomplish this transformation. The reaction of the acid chloride and the amine is best accomplished at a temperature from about 0-25°C employing an excess of the amine and a non-reactive solvent such as acetone, benzene, and the like. To make the primary amide, ammonium hydroxide and a water miscible solvent such as acetone are preferably employed. Heating the reaction mixture is not required but will facilitate the reaction.

The primary amide (III, $R_3 = R_4 =$ hydrogen) can be used as an intermediate to the corresponding nitrile IV. This transformation is best accomplished by treating the nitrile with triphenylphosphine, tetrahydrofuran, and carbon tetrachloride at a temperature from about 45-55°C following the procedure of Yamato et al., Tetrahedron Letters, 4353 (1970).

Nitrile IV can be used to prepare tetrazole V. Standard methods of transforming nitriles into tetrazoles may be employed. Preferably, three equivalents of sodium azide and three equivalents of ammonium chloride are allowed to react with the nitrile in a solvent such as dimethylformamide at a temperature from about 100-120°C.

Preparation of the methanol derivative VI may be accomplished by standard reductive techniques. The preferred method involves the lithium aluminum hydride reduction of the acid II in tetrahydrofuran.

The intermediate compounds of Formula II wherein Z is hydrogen are taught in U.S. Patent No. 4,552,891 and references cited therein. The 3-methyl analogue (i.e. Z is methyl) can be prepared in analogous fashion.

The compounds employed in the claimed invention may be administered by various routes including the oral, rectal, transdermal, subcutaneous, intravenous, intramuscular, inhalation, or intranasal routes, being usually employed in the form of a pharmaceutical composition. Such compositions are prepared in a manner well known in the pharmaceutical art and comprise at least one active compound. Accordingly, the invention includes a pharmaceutical composition comprising as active ingredient about 5 to about 500 mg of a compound of Formula I or a pharmaceutically acceptable salt thereof associated with a pharmaceutically acceptable carrier.

In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a

vehicle, excipient or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propyl-hydroxybenzoates, talc, magnesium stearate or mineral oil. The formulations can additionally include lubricating agents, wetting agents, emulsifying and suspending agents, preserving agents, sweetening agents or flavoring agents. The compositions of the invention may, as is well known in the art, be formulated so as to provide quick, sustained or delayed release of the active ingredient after administration to the patient.

The compositions are preferably formulated in a unit dosage form, each dosage containing from about 5 to about 500 mg (from about 5 to 50 mg in the case of parenteral or inhalation administration, and from about 25 to 500 mg in the case of oral or rectal administration) of a compound of formula I. The term "unit dosage form" refers to physically discrete units suitable as unitary dosages for human subjects and other mammals, each unit containing a predetermined quantity of active material calculated to produce the desired therapeutic effect, in association with the required pharmaceutical carrier.

The active compounds are effective over a wide dosage range. For example, dosages per day will normally fall within the range of about 0.5 to about 300 mg/kg. In the treatment of adult humans, the range of about 1 to about 50 mg/kg, in single or divided doses, is preferred. However, it will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms, and therefore the above dosage ranges are not intended to limit the scope of the invention in any way.

Preferred pharmaceutical forms of the present invention are capsules, tablets, suppositories, injectable solutions, creams and ointments. Especially preferred are formulations for inhalation or insufflation application, such as an aerosol, and for oral ingestion.

The following examples further illustrate the preparation of the compounds used in this invention. The examples are illustrative only and are not intended to limit the scope of the invention in any way.

## Example 1

5,6-Diethoxybenzo[b]thiophene-2-carboxamide

One-half gram of 5,6-diethoxybenzo[b]thiophene-2-carboxylic acid was heated for one hour at reflux in 8 ml of freshly distilled thionyl chloride. The solution was concentrated to dryness in vacuo. The residue was dissolved in 20-25 ml of acetone and added dropwise to approximately 50 ml of concentrated ammonium hydroxide which was previously chilled to 0° C. After stirring for 1 hour, the mixture was concentrated in vacuo to remove the acetone and the resulting crystals were collected by filtration providing 447.5 mg of the desired title product, m.p. = 191.5-192.5.

Analysis for $C_{13}H_{15}NO_3S$:
Calculated: C, 58.85; H, 5.70; N, 5.28; S, 12.08;
Found: C, 58.66; H, 5.58; N, 4.99; S, 12.03.

## Example 2

1-[(5,6-Diethoxybenzo[b]thien-2-yl)carbonyl]-4-methylpiperazine

Following the procedure of Campaigne et al., J. Med. Chem., 10, 945 (1967), 0.803 g of 5,6-diethoxybenzo[b]thiophene-2-carboxylic acid chloride, 0.989 g of N-methylpiperazine, 51.95 ml of water, and 4.1 ml of 10% sodium hydroxide were stirred overnight at room temperature. The title product was recovered in 76% yield by filtration.

Analysis for $C_{18}H_{24}N_2O_3S$:
Calculated: C, 62.04; H, 6.94; N, 8.04;
Found: C, 61.92; H, 6.96; N, 7.88.

## Example 3

N,N-Diethyl-5,6-diethoxybenzo[b]thiophene-2-carboxamide

A mixture of 0.803 g of 5,6-diethoxybenzo[b]thiophene-2-carboxylic acid chloride, 0.206 g of diethylamine, and 25 ml of benzene was heated at reflux for approximately 16 hours. The mixture was concentrated in vacuo. The residue was purified by preparative thin layer chromatography eluting with 8:4.2:0.3 hexane/ethyl acetate/acetic acid affording 0.148 g of the title product.

Analysis for $C_{17}H_{23}NO_3S$:
Calculated: C, 63.52; H, 7.21; N, 4.36;
Found: C, 61.11; H, 7.34; N, 3.71.

Example 4

5,6-Diethoxy-N-1H-tetrazol-5-ylbenzo[b]thiophene-2-carboxamide

A mixture of 5.0 g of 5,6-diethoxybenzo[b]thiophene-2-carboxylic acid and 3.36 g of 1,1′-carbonyldiimidazole was stirred for 1.5 hours in 80 ml of dimethylformamide. Dry 5-aminotetrazole monohydrate (2.52 g.) was added and the mixture was heated to 80° C for approximately 18 hours. The reaction mixture was poured into water and acidified with aqueous hydrochloric acid. The resulting precipitate was recovered by filtration. The solid was triturated twice with boiling ethyl acetate to provide 3.39 g of desired title product, m.p. >260° C.

Analysis for $C_{14}H_{15}N_5O_3S$:

Calculated: C, 50.44; H, 4.54; N, 21.01; S, 9.62;

Found: C, 50.14; H, 4.47; N, 20.82; S, 8.79.

Example 5

5,6-Diethoxybenzo[b]thiophene-2-carbonitrile

A suspension of 250 mg of 5,6-diethoxybenzo[b]thiophene-2-carboxamide in 10 ml of tetrahydrofuran was added to a mixture of 493 mg of triphenylphosphine in 10 ml of carbon tetrachloride. The reaction was stirred at 50° C for 24 hours. The mixture was allowed to cool to ambient temperature, the suspended solid was removed by filtration, and the filtrate was concentrated in vacuo to yield a pale gold solid. Preparative thin layer chromatography employing 8:4.2:0.3 hexane/ethyl acetate/acetic acid provided 82.5 mg of the desired title product, m.p. 105.5-106.5° C.

Analysis for $C_{13}H_{13}NO_2S$:

Calculated: C, 63.15; H, 5.30; N, 5.66;

Found: C, 63.56; H, 5.53; N, 5.09.

Example 6

5-(5,6-Diethoxybenzo[b]thien-2-yl)-1H-tetrazole

A mixture of 1.0 g of 5,6-diethoxybenzo[b]thiophene-2-carbonitrile, 786 mg of sodium azide, and 647 mg of ammonium chloride was stirred for 20 hours at 120° C in 25 ml of dimethylformamide. The mixture was added to aqueous hydrochloric acid and filtered. The solid was dissolved in ethyl acetate and extracted into 10% aqueous sodium carbonate. The basic solution was then acidified with aqueous hydrochloric acid and filtered to provide 534.6 mg of the title product, m.p. 197-200° C.

Analysis for $C_{13}H_{14}N_4O_2S$:

Calculated: C, 53.78; H, 4.86; N, 19.30; S, 11.04;

Found: C, 53.51; H, 4.59; N, 19.08; S, 10.99.

Example 7

5,6-Diethoxybenzo[b]thiophene-2-methanol

A solution of 10.0 g of 5,6-diethoxybenzo[b]thiophene-2-carboxylic acid in 200 ml of tetrahydrofuran was added to a slurry of 1.8 g of lithium aluminum hydride in 150 ml of dry tetrahydrofuran, cooled to 0° C. The resulting mixture was allowed to stir overnight under a nitrogen blanket at room temperature. The reaction mixture was cooled to 0° C and treated with diethyl ether and aqueous sodium hydroxide. The layers were separated, and the organic layer was dried and concentrated in vacuo to provide 9.42 g of the desired title product, m.p. = 80-82° C.

Analysis for $C_{12}H_{16}O_3S$:

Calculated: C, 61.88; H, 6.39; S, 12.71;

Found: C, 61.67; H, 6.47; S, 12.75.

The following formulation examples may employ as active compounds any of the pharmaceutical compounds defined above.

Example 8

Hard gelatin capsules are prepared using the following ingredients:

| | Quantity (mg/capsule) |
|---|---|
| 5,6-Dimethoxybenzo[b]thiophene-2-carboxamide | 250 |
| Starch | 200 |
| Magnesium stearate | 10 |

The above ingredients are mixed and filled into hard gelatin capsules in 460 mg quantities.

Example 9

A tablet is prepared using the ingredients below:

| | Quantity (mg/tablet) |
|---|---|
| 5,6-Dipropoxybenzo[b]thiophene-2-methanol | 250 |
| Cellulose, microcrystalline | 400 |
| Silicon dioxide, fumed | 10 |
| Magnesium stearate | 5 |

The components are blended and compressed to form tablets each weighing 665 mg.

Example 10

An aerosol solution is prepared containing the following components:

| | Weight % |
|---|---|
| 5-Ethoxy-6-methoxybenzo[b]thiophene-2-carbonitrile | 0.25 |
| Ethanol | 30.00 |
| Propellant 11 (trichlorofluoromethane) | 10.25 |
| Propellant 12 (Dichlorodifluoromethane) | 29.75 |
| Propellant 114 (Dichlorotetrafluoroethane) | 29.75 |

The active compound is dissolved in the ethanol and the solution is added to the propellant 11, cooled to -30°C. and transferred to a filling device. The required amount is then fed to a container and further filled with the pre-mixed propellants 12 and 114 by means of the cold-filled method or pressure-filled method. The valve units are then fitted to the container.

Example 11

Tablets each containing 60 mg of active ingredient are made up as follows:

| | |
|---|---|
| N-Methyl-N-propyl-5-methoxy-6-isopropoxy-benzo[b]thiophene-2-carboxamide | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C. and passed through a No. 18 mesh U.S. sieve. The sodium carboxymethyl starch, mag nesium stearate and talc, previously passed through a No. 60 mesh U.S. sieve, are then added to the granules which, after mixing, are compressed on a tablet machine to yield tablets each weighing 150 mg.

Example 12

Capsules each containing 80 mg of medicament are made as follows:

| | |
|---|---|
| 5,6-Diethoxybenzo[b]thiophene-2-carboxamide | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Example 13

Suppositories each containing 225 mg of active ingredient are made as follows:

5,6-Dimethoxy-N-1H-tetrazol-5-ylbenzo[b]thiophene-2-carboxamide 225 mg
Unsaturated or saturated fatty acid glycerides to 2,000 mg

The active ingredient is passed through a No. 60 mesh U.S. sieve and suspended in the fatty acid glycerides previously melted using the minimum heat necessary. The mixture is then poured into a suppository mold of nominal 2 g capacity and allowed to cool.

Example 14

Suspensions each containing 50 mg of medicament per 5 ml dose are made as follows:

5-(6-Ethoxy-5-propoxybenzo[b]thien-2-yl)-1H-tetrazole 50 mg
Sodium carboxymethyl cellulose 50 mg
Sugar 1 g
Methyl paraben 0.05 mg
Propyl paraben 0.03 mg
Flavor q.v.
Color q.v.
Purified water to 5 ml

The medicament is passed through a No. 45 mesh U.S. sieve and mixed with the sodium carboxymethylcellulose, sugar, and a portion of the water to form a suspension. The parabens, flavor and color are dissolved and diluted with some of the water and added, with stirring. Sufficient water is then added to

produce the required volume.

The compounds described herein and their pharmaceutically acceptable salts possess useful pharmaceutical properties. The compounds are useful as inhibitors of the enzyme phosphodiesterase, and are also useful in blocking anaphylactic responses and bronchoconstriction. The compounds are therefore useful in treating mammals suffering from or susceptible to asthma. This biological activity was demonstrated in the following test system.

Following the general procedure taught by Andersson, Brit. J. Pharmacol., 69, 467 (1980), mixed sex Hartley strain guinea pigs (250-300 g) were sensitized to ovalbumin by a single injection of 1 mcg of ovalbumin mixed with 50 mg of aluminum hydroxide per animal. These animals were used 21 to 26 days later for aerosol challenge with ovalbumin.

Anaphylaxis was induced by exposure to an aerosol of 10 mg of ovalbumin per milliliter of water for 10 minutes delivered by a Tri-R venturi nebulizer, particle size 2 to 5 micron diameter, at a delivery rate of 0.4 ml of solution per minute. The guinea pigs were placed in specially constructed chamber for exposure to the aerosol. The aerosol was introduced from the bottom of the chamber and exhausted at the top. A baffle above the chamber inlet port provided even distribution of the aerosol throughout the chamber. The animals were supported above the inlet on a wire mesh disc. The chamber was under slight negative pressure to enhance the exhaust of the aerosol which was passed through a calcium chloride trap followed by a super-cooled condenser trap.

Groups of five guinea pigs were treated with the compounds of this invention, clinical anti-asthmatic agents, or placebo (vehicle only) two hours prior to the aerosol challenge. All the animals also received 3 mg/kg pyrillamine orally two hours prior to the aerosol challenge in order to blunt the histamine component of the anaphylaxis. The compounds were administered orally as a suspension in 10% acacia or intraperitoneally in 10% acacia.

Throughout the ten minute period of aerosol challenge, the animals were observed for the symptomology of convulsive cough, convulsive collapse, and death as described by Herxheimer, J. Physiology, 117, 251 (1952). The number of animals that responded to the aerosol challenge for each of the above parameters as well as the time for the response to occur were recorded. The data were analyzed by comparing the severity index (the sum of the number of animals that coughed, collapsed, and/or died) between the treated and placebo group of guinea pigs. Thus, the maximum severity index for each group of five guinea pigs is 15. On the average, the severity index for the placebo group was 10-12. The percent inhibition of anaphylaxis was determined by the following formula:

$$\%\ \text{inhibition} = \left[1 - \frac{S_d}{S_p}\right] \times 100$$

where $S_d$ is the severity index for the drug treated animals and $S_p$ is the severity index of the placebo treated animals. The results of these experiments are summarized in Table 1.

Table 1

Antigen induced anaphylaxis in guinea pigs

| Compound of Example No. | Dose mg/kg | Route of Administration | Percent Inhibition |
|---|---|---|---|
| 3 | 50 | P.O. | 38% |
| | 50 | I.P. | 82% |
| | 30 | I.P. | 89% |
| | 10 | I.P. | 44% |
| 4 | 50 | P.O. | 56% |
| 5 | 50 | P.O. | 22% |
| | 50 | I.P. | 74-84% |
| | 30 | I.P. | 69% |
| 6 | 50 | P.O. | 43% |
| 7 | 50 | P.O. | 68% |
| | 50 | I.P. | 74% |
| | 30 | I.P. | 89% |

**Claims**

1. A compound of the formula

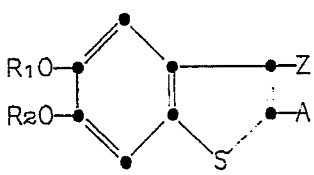

I

wherein each of $R_1$ and $R_2$ is independently $C_1$-$C_3$ alkyl, Z is hydrogen or methyl, and A is $-CH_2OH$, -CN, 5-tetrazolyl, or $-CONR_3R_4$, where $R_3$ and $R_4$ are each independently hydrogen or $C_1$-$C_3$ alkyl, or when one of $R_3$ and $R_4$ is hydrogen, the other of $R_3$ and $R_4$ is 5-tetrazolyl, or when taken together with the nitrogen atom to which they are attached form a piperidino, morpholino, or N-methyl piperazino ring, or a pharmaceutically acceptable salt thereof; for use as a bronchodilator.

2. A compound for use as a bronchodilator according to claim 1 employing a compound wherein Z is hydrogen.

3. A compound for use as a bronchodilator according to claim 1 or 2 employing a compound wherein $R_1$ and $R_2$ are each ethyl.

4. A compound for use as a bronchodilator according to claim 1 wherein the compound is 5,6-diethoxybenzothiophene-2-carboxamide.

5. A compound for use as a bronchodilator according to claim 1 wherein the compound is 5,6-diethoxybenzothiophene-2-methanol.

6. A compound as defined in any one of claims 1 to 5, for use in the therapy of asthma.

7. A pharmaceutical formulation comprising a compound of the formula I as defined in any one of claims 1 to 5,

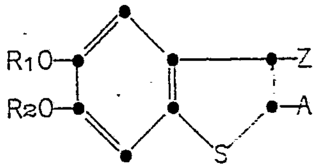

I

associated with one or more pharmaceutically acceptable carriers or diluents therefor.

8. A method of preparing a pharmaceutical formulation which comprises admixing a compound of Formula I as defined in any one of claims 1 to 5 with one or more pharmaceutically acceptable carriers or dilutents.